# EUROPÄISCHE PATENTSCHRIFT

(11) **EP 0 654 248 B1**
(45) Veröffentlichungstag und Bekanntmachung des Hinweises auf die Patenterteilung: **02.04.1997**
(21) Anmeldenummer: 94118281.8
(22) Anmeldetag: 21.11.1994
(51) Int. Cl.: A61B 17/58

(54) **Kraftübertragungsglied für Osteosynthesearbeiten**
Load transfer member for osteosynthesis
Elément de transfert de charge pour l'ostéosynthèse

(30) Priorität: 23.11.1993 DE 4339804
(43) Veröffentlichungstag der Anmeldung: 24.05.1995
(73) Patentinhaber: Härle, Anton, Prof. Dr., D-48161 Münster (DE)
(72) Erfinder: Härle, Anton, Prof. Dr., D-48161 Münster (DE)

(56) Entgegenhaltungen:
- EP-A- 0 472 017
- EP-A- 0 486 762
- DE-A- 3 630 863

## Beschreibung

Die Erfindung betrifft Kraftübertragungsglieder für Osteoynthesearbeiten, wie Schrauben, Schraubeinsätze, Stifte, Osteosyntheseplatten, stabförmige Lastträger, Prothesenkomponenten, in den Knochen eingreifende Operationsinstrumente.

Zum besseren Verständnis wird in der folgenden Beschreibung eine begriffliche Unterteilung in der Art vorgenommen, daR die Kraftübertragungsglieder, die direkt in den Knochen eingreifende Implantate darstellen, als **Knochenfixationsmittel**, die nicht in den Knochen eingreifenden Implantate als **Lastträger** und die nur während der Operationszeit verwendeten Kraftübertragungsglieder als **Instrumente**, wie Knochengewindebohrer, flexible Wellen, bezeichnet werden.

Im Folgenden wird die zugrunde liegende Problematik und das erfindungsgemäße Lösungsprinzip hauptsächlich anhand der Knochenfixationsmittel und Lastträger abgehandelt.

Knochenfixationsmittel, wie Knochenschrauben bzw. Stifte mit Knochengewindeanteil werden meist in Kombination mit Lastträgern, wie Platten-, Stabsystemen, angewandt, um Knochen bzw. Knochenabschnitte in eine bestimmten Stellung zueinander auszurichten und/oder in dieser zu fixieren.

Bei der herkömmlichen Osteosynthese an Röhrenknochen wird dabei eine mit Löchern versehene Platte mittels durch diese hindurchgreifender Knochenschrauben am Knochen fixiert. Die Ruhigstellung der Knochen bzw. Knochenteile erfolgt durch Anpressen an die Platte mittels Schrauben, wobei die Schraubenköpfe auf der dem Knochen gegenüberliegenden Seite in den sphärischen Abschnitten der Schraubenlöcher ein Widerlager finden.

Die stabile Verbindung der Knochen bzw. Knochenteile wird also über eine Anpressung von Schraubenköpfen an die Knochenplatte und über die sekundär damit hervorgerufene Anpressung der Knochenplatte an den Knochen selbst erreicht. Die im Knochen verankerten Knochenschrauben werden in ihrem Verlauf im Knochen selbst praktisch nur auf Zug beansprucht, während in diesem Bereich die Biegekräfte durch das Knochengewebe aufgefangen und abgefedert werden. Am Austritt der Schrauben aus dem Knochen bzw. bei Verlust des Kontakt mit der harten Knochenrinde, wie z.B. im Wirbelbogenabschnitt, treten dann wesentlich größere, mechanische Beanspruchungen auf, wobei insbesondere Biege- und Scherbeanspruchungen auf die Schraubenabschnitte einwirken, die zwischen Knochenoberfläche und Kopfauflage an der Platte liegen.

Dieser Abschnitt herkömmlicher Schrauben ist daher einer maximalen Belastung unterworfen, wobei das hier eingeschnittene Gewinde noch eine zusätzliche Materialschwächung und Kerbwirkung erzeugt.

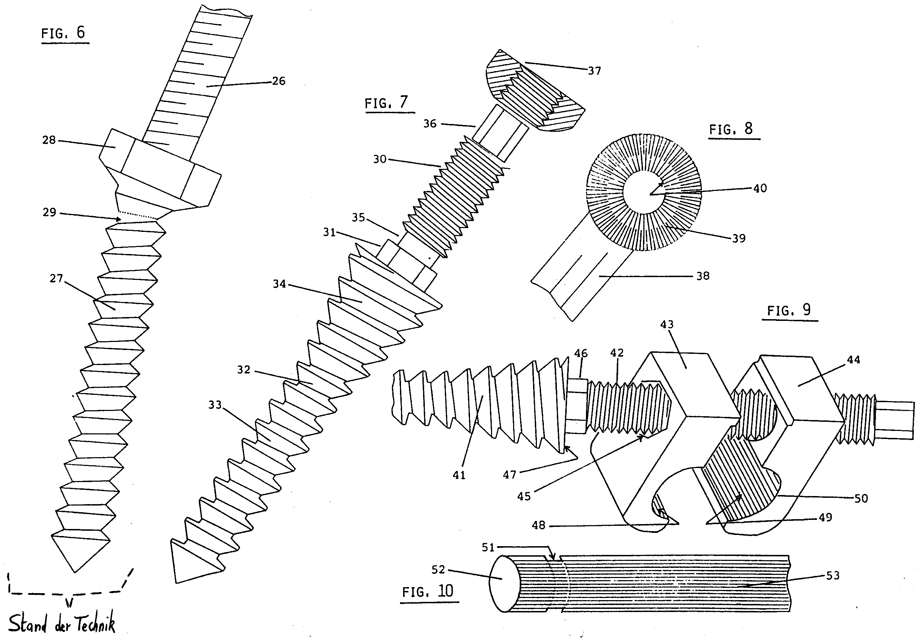

In der klinischen Praxis sind daher immer wieder Schraubenbrüche zu beobachten, die in typischer Weise an dieser Prädilektionsstelle auftreten. Da der im Knochen verbleibende, abgebrochene Schraubenanteil praktisch mit der Knochenoberfläche abschließt (11 in Fig. 2) und keine Angriffsfläche bietet, müssen zu seiner Entfernung mittels Hohlbohrer relativ große Knochenaufbohrungen vorgenommen werden, damit dann mit einer Zange oder einem Konus die Schraube im Gewindeabschnitt gefaßt und herausgedreht werden kann.
Diese Prozedur führt zu unerwünschten und beträchtlichen Schwächungen der Knochenstabilität, sodaß einerseits Knochenbruchgefahr besteht und andererseits die Notwendigkeit zur Übertragung von Knochengewebe mit dem Ziel der Auffüllung des so geschaffenen Knochendefekts gegeben sein kann.

Bei Fixationsverfahren an der Wirbelsäule ist die Beanspruchung der mittels Schraubgewinden in den Wirbelkörpern verankerten Knochenfixationsmittel, wie Pedikelschrauben oder Gewindestiften am Austritt aus dem Knochen noch größer, da aufgrund der anatomischen Verhältnisse jeder Knochen nur durch ein Knochenfixationsmittel mit dem axialen Lastträger, sei es eine Platte oder ein Stab, verbunden werden kann. Eine weiteres Belastungsmoment entsteht durch den relativ langen Hebel im Kraftübertragungsglied, da im Gegensatz zu den Verhältnissen beim Röhrenknochen das Kraftübertragungsglied an der Wirbelsäule bis zu 2 cm nach dorsal aus dem Knochen herausragt, bis es mit dem axialen Lastträger verkoppelt ist.

Ein weiteres Stabilitätsproblem resultiert daraus, daß die Platten oder Stäbe nicht breitflächig an Knochen-Kontaktflächen angepreßt werden können, und so nicht einen Teil der Biegebeanspruchung in eine Zugbelastung umwandeln können.

Brüche der Fixationselemente an der Wirbelsäule sind daher viel häufiger als an den Röhrenknochen und betragen rund 10 %. Die Entfernung der abgebrochen, im Knochen verbliebenen gewindetragenden Abschnitte stellt ein noch größeres Problem als bei den Röhrenknochen dar. Es muß dann meist ein wesentlicher Teil der, wenn nicht die ganze Bogenwurzel entfernt werden, in der die Schraube hauptsächlich ihre Verankerung erlangt; eine nochmalige Fixation dieses Wirbelsäulensegmentes ist danach dann nahezu ausgeschlossen.

Wegen des erheblichen Gefährdungspotentials des Rückenmarks, das nur rund 1-2 mm neben dem zu entfernenden Knochenabschnitt liegt, wird auf die Entfernung abgebrochener Schrauben an der Wirbelsäule häufig verzichtet, was wiederum ein nicht zu vernachlässigendes Dauerrisiko beinhaltet.

Aus dem Dokument DE-A 3630863 ist eine die Merkmale des Oberbegriffs des unabhängigen Anspruchs 2 aufweisende Knochenschraube bekannt, die ein Element zur Drehmomentübertragung im Bereich des Kopfes aufweist, wobei das Element auf der dem Knochengewinde gegenüberliegenden Seite der Schraube als Fortsatz des Schraubenkopfes ausgebildet und vom Schraubenkopf durch eine Sollbruchstelle getrennt ist. Beim Überschreiten eines bestimmten Drehmomentes während der Schraubenmontage kommt es dann im Bereich der Sollbruchstelle zu einem Abdrehen des Fortsatzes. Der geringere Querschnitt im Bereich der Sollbruchstelle wird dabei zur Einhaltung eines bestimmten Drehmomentes benutzt.
Das Dokument DE-A-3630863 befaßt sich mit einer Drehmomentsteuerung bzw. -begrenzung bei der Montage von Knochenschrauben.

Der Erfindung liegt daher die Aufgabe zugrunde, Kraftübertragungsglieder für osteosynthetische Arbeiten zu schaffen, die so ausgelegt sind, daß auch beim Bruch des Kraftübertragungsglieds eine leichte Entfernbarkeit gewährleistet ist.

Diese der Erfindung zugrundeliegende Aufgabe wird für instrumentartige Kraftübertragungsglieder durch die Lehre des unabhängigen Anspruchs 1, und für Knochenfixationsmittel oder lasträgerartige Kraftübertragungsglieder durch die im unabhängigen Anspruch 2 angegebenen Merkmale gelöst.

Vorteilhafte Ausgestaltungen sind in den Unteransprüchen erläutert.

Mit anderen Worten ausgedrückt wird vorgeschlagen, Kraftübertragungsglieder mit einer Sollbruchstelle auszurüsten, an der es bei Überlastung des osteosynthetischen Implantats oder Instrumentes zuerst zum Bruch kommt, wobei die Sollbruchstelle so lokalisiert ist, daß eine leichte Entfernbarkeit der gesamten Implantate oder des ganzen kraftübertragenden Operationsinstrumentes sichergestellt ist. Die Sollbruchstelle kann an den Knochenfixationsmitteln, wie Schraube oder Gewindestift, an deren Übergang vom Knochengewindebereich zur Ankopplungsvoorichtung lokalisiert sein, sodaß beim Bruch des Knochenfixationsmittels an der vorbestimmten Stelle, d.h. außerhalb des Knochens die Handhabe oder zumindest ein funktionsfähiger Teil der Handhabe am im Knochen liegenden Teil des Knochenfixationsmittels verbleibt. Die Sollbruchstelle kann auch am Lastträger lokalisiert sein, indem dieser an seiner Sollbruchstelle das schwächste Glied der Implantatkomponenten darstellt.

Bei in den Knochen eingreifenden Operationsinstrumenten ist die Sollbruchstelle so zu plazieren, daß z.B. ein unter Rotationskraftüberlastung auftretender Bruch des Knochengewindebohrers vor seinem Eintritt in den Knochen auftritt, und nach dem Bruch ein mit einem Betätigungswerkzeug faßbarer Bereich außerhalb des Knochens zur Verfügung steht.

Damit kann nun der abgebrochene Teil des Kraftübertragungsglieds gefaßt und/oder mittels der daran enthaltenen Handhabe problemlos aus dem Knochen herausgedreht und somit ohne Knochenresektion entfernt werden.

Die Sollbruchstelle kann dabei eine Einkerbung, ringförmige Querschnittsreduzierung oder materialmäßige Schwächung darstellen. Die Sollbruchstelle muß so am Kraftübertragungsglied plaziert sein, daß bei Überlastung des Kraftübertragungsglieds in ihr angesichts der gegebenen Belastungsverhältnisse zuerst der Bruch erfolgt.

Weiterhin wird eine Verstärkung des die Handhabe enthaltenden Schaftanteils und des an die Handhabe anschließenden, endständigen Knochengewindeabschnittes vorgeschlagen.

Bei Rundkopfschrauben zur Verwendung mit Osteosyntheseplatten wird weiterhin ein gewindefreier Abschnitt zwischen Handhabe und Knochengewinde vorgeschlagen, d.h. daß der aus dem Knochen herausragende und im Schraubenloch liegende Schraubenabschnitt eine Verstärkung erfährt. Die Sollbruchstelle ist hier am Schaftübergang zum Rundkopf anzubringen, wobei dann im Schaft selbst die imbusartige Handhabe über diese Sollbruchstelle hinaus in Richtung des Gewindeabschnittes verlängert ist.

Bei Knochenfixationsmitteln an der Wirbelsäule kann sich die Handhabe an den Verstärkungsteil anschließen und die Sollbruchstelle jenseits der Handhabe befinden. Die Sollbruchstelle kann dabei durch eine Querschnittsverringerung z.B. des der Ankopplung an ostesynthetische Lastträger dienenden Maschinengewindes (ISO oder ähnliches Gewindeprofil) gewährleistet sein, wobei der Kerndurchmesser des Maschinengewindes die relevante Größe darstellt, oder durch Einkerbungen oder ringförmige Einschnürungen des Schaftanteils jenseits der Handhabe.

An den Kraftübertragungsgliedern kann die Sollbruchstelle primär als Einkerbung, Querschnittsreduzierung oder materialmäßige Schwächung ausgelegt sei, oder sich erst unter der Montage als eine durch den Eingriff von Zusatzteilen, wie von Klammerkanten hervorgerufene Einbuchtungen ausbilden. Für die Ausbildung einer erwünschten Sollbruchstelle in einem bestimmten Element eines komplexen Implantatsystem vermag angesichts der Gesamtbelastungsverhältnisse auch schon die Unterdimensionierung eines Kraftübertragungsgliedes ausreichend sein, wenn es sich bei dem zuerst brechenden Kraftübertragungsglied z.B. um einen Lastträger handelt, der leicht, d.h. ohne wesentlichen Operationsaufwand und ohne Knochenresektion entfernbar ist.

Bei stabförmigen Lastträgern kann die angestrebte Sollbruchstelle durch die angesichts der Belastungsvefhältnisse bewußt ausgewählte Unterdimensierung des Stabquerschnitts, oder durch segmentale Querschnittsreduzierungen primär eingerichtet sein, oder sich aus montagebedingten Einkerbungen sekundär ausbilden.

Bei Osteosyntheseplatten kann die Einrichtung einer Sollbruchstelle durch Querschnittsreduzierung an kritischen Bereichen, wie Schraubenlöchern z.B. durch eine Vergrößerung des Öffnungswinkels (25 in Fig. 5) erreicht werden, wobei durch die Vergrößerung der Durchtrittsöffnung nun auch sichergestellt wird, daß der gebrochene Schraubenschaft durch diese hindurch gleiten kann und es nicht zu Sekundärbrüchen im Knochengewindeabschnitt der Schraube kommt.

Die der Ankopplung an eine osteosynthetische Lastträger dienende Ankopplungseinrichtung kann direkt am Schaft des Knochenfixationsmittels an die Handhabe anschließend ausgebildet sein oder sich aus mit dem Knochenfixationsmittel zusammenwirkenden Zusatzteilen, wie z.B. Klammern zusammensetzen.

Die Auflagefläche für derartige Zusatzteile oder der Lastträger selbst kann rotationssichernde Ausgestaltungen ausweisen, wie Riffelungen, Rasterungen, nicht-runde Außenform.

Die Stabilität eines Knochenimplantats aus mehreren Komponenten kann weiter verbessert werden, wenn die Knochenfixationsmittel die maximalen Außendurchmesser aufweisen, die angesichts der anatomischen Verhältnisse möglich sind. Bei der besonders kritischen Wirbelsäulenfixation ließe sich durch eine größere Dimensionierung der in den Knochen eingreifenden Fixationsmittel bei unveränderter, aber angemessener Dimensionierung der Lastträger der Überlastungsbruch automatisch aus dem Knochenbereich heraus verlagern. Diese optimale Größendimensionierung erfordert aber angesichts der besonderen Operationsbedingungen ein spezielles Gewindeprofil, das ein sukzessives Ansteigen des Außendurchmessers bei Gewindeherstellungswerkzeugen wie auch Knochenfixationsmitteln erlaubt, ohne daß dadurch das Knochengewinde wesentlich geschwächt oder gar zerstört wird. Dazu ist z.B. erforderlich, daß die Gewindesteigung (h) auch bei unterschiedlichen Außen- und/oder Kerndurchmesser, wie auch unterschiedlichen Gewindetiefen (gt) konstant ist.

Diese Forderung steht im Widerspruch zu dem herkömmlichen Gewindestandard, der einen konstanten Flankenwinkel vorschreibt.

In einer weiteren Ausführungsform wird daher für die gewindetragenden Körper ein abgeändertes Gewindeprofil vorgeschlagen, bei dem innerhalb eines Satzes zusammengehörender, gewindetragender Körper die Gewindesteigung (h) konstant ist, dagegen die Außendurchmesser, Kerndurchmesser, Gewindetiefen und Flankenwinkel variabel sind.

Ausführungsbeispiele des Standes der Technik und zweier erfindungsgemäßen Schraubenausgestaltungen und der dazugehörenden Ankopplungseinrichtungen, sowie zweier Lastträger gemäß der Erfindung werden nachfolgend anhand der Zeichnungen erläutert.

Die Zeichnungen zeigen dabei in:
- Fig. 1: eine Rundkopfknochenschraube des Standes der Technik, in
- Fig. 2: eine abgebrochene Schraube des Technikstandes, in
- Fig. 3: eine Rundkopfknochenschraube mit Verdickung des außerhalb des Knochen liegenden Schraubenanteils und einer erfindungsgemäßen Sollbruchstelle am Übergang des Schaftes zum Rundkopf, wobei diese auch durch die in den Schaft hineinreichende Imbus-Handhabe hervorgerufen wird, in
- Fig. 4: einen Zustand nach Bruch der erfindungsgemäßen Knochenschraube, in
- Fig. 5: ein Rundloch einer Osteosyntheseplatte zur Aufnahme einer erfindungsgemäßen Knochenschraube, in
- Fig. 6: ein gebrochenes Wirbelsäulen-Fixationmittels des Standes der Technik, in
- Fig. 7: eine erfindungsgemäße Ausführungsform eines Wirbelsäulen-Fixationmittels mit Verstärkungsteil des endständigen Knochengewindeabschnitts, einer diesem benachbarten Handhabe und des daran anschließenden Bereichs mit der Sollbruchstelle, in
- Fig. 8: eine Ankopplungseinrichtung, die in den Schaft integriert ist, in
- Fig. 9: eine zweiteilige Klammer, die zusammen mit dem Maschinengewindeabschnitt des Knochenfixationmittels eine Ankopplungseinrichtung für einen Lastträger bildet, in
- Fig. 10: einen stabförmigen Lastträger mit segmentaler Querschnittsreduzierung und rotationsichernder Längsrillung auf der Außenkontur, in
- Fig. 11: ein spezielles Gewindeprofil für die gewindetragenden Körper mit konstanter Gewindesteigung bei unterschiedlichen Kern- u. Außendurchmessern.

Ausführungsbeispiele zweier erfindungsgemäßen Schraubenausgestaltungen und der dazugehörenden Ankopplungseinrichtungen, sowie zweier Lastträger gemäß der Erfindung werden nachfolgend anhand der Zeichnungen erläutert. Die Zeichnungen zeigen dabei:

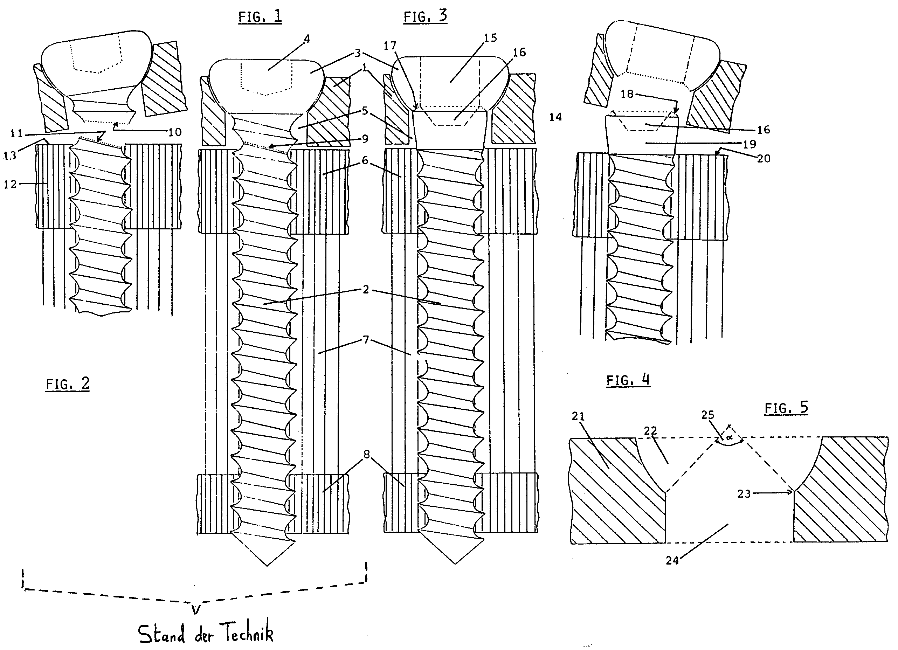

- In Fig. 1: ist eine übliche, zum Stand der Technik gehörende Rundkopfknochenschraube zur Verwendung mit einer Osteosyntheseplatte 1 dargestellt, die einen Knochengewindeabschnitt 2, einen gerundeten Schraubenkopf 3 und eine in den Schraubenkopf hineinreichende imbusartige Handhabe 4 aufweist. Der gerundete Schraubenkopf liegt in einem in Komplementärform ausgestalteten Schraubenloch 5 der Osteosynthesplatte; die Schraube durchsetzt mit ihrem Knochengewindeabschnitt die anliegende Knochenrinde 6, den spongiösen Markraum 7 und die gegenüberliegende zweite Knochenrinde 8. Als punktierte Linie 9 ist der für Schraubenbrüche kritische Bereich im zweiten Gewindegang gekennzeichnet, in dem es meist zum Bruch kommt.
- In Fig. 2: ist ein Teil der Knochenschraube nach Fig. 1 mit etwas abgehobener Osteosyntheseplatte und den anliegenden Knochenabschnitten nach Schraubenbruch dargestellt. Mit 10 ist die Bruchkante des mit dem Schraubenkopf zusammenhängenden Segmentes, mit 11 die Bruchkante des im Knochen 12 verbleibenden Schraubenteils bezeichnet; das im Knochen liegende Schraubensegment überragt die Knochenoberfläche 13 kaum und kann daher nicht gefaßt werden.
- In Fig. 3: ist ein Ausführungsbeispiel der erfindungsgemäßen Rundkopfknochenschraube dargestellt, wobei die mit den Zahlen 1, 2, 3, 5, 6, 7 und 8 bezeichneten Bereichen den Verhältnissen in Fig. 1 entsprechen. Mit 14 ist der gewindelose und sich konisch verstärkende Abschnitt der Knochenschraube dargestellt, der außerhalb des Knochens liegt und in das Schraubenloch hineinragt. Mit 15 ist ein erstes Segment der imbus-artigen Handhabe 15 bezeichnet, das im Querdurchmesser und in seiner Längsausgestaltung vergrößert und dem Angriff eines Schraubenziehers dient. Mit 16 ist ein zweites imbusartiges Segment der Handhabe bezeichnet, das konisch gestaltet ist und an seinem Übergang zum ersten Segment 15 die Sollbruchstelle 17 darstellende Materialschwächung ausbildet.
- In Fig. 4: ist ein Teil der Knochenschraube nach Fig. 3 mit etwas abgehobener Platte und den anliegenden Knochenabschnitten nach Schraubenbruch dargestellt. Mit 17 ist die Bruchkante im Schraubenkopf bezeichnet; das erste Segment 15 der imbus-artigen Handhabe stellt nun einen durchgehenden Kanal dar. Mit 18 ist die Bruchkante des in den Knochen hineinragenden Schraubenteils bezeichnet, die das in diesem Schraubenabschnitt verbleibende zweite imbusartige Handhabe-Segment 16 umgibt. Durch Eingriff eines Betätigungswerkzeuges, wie eines Schraubenziehers, in dieses zweite Handhabesegment kann nun auch eine abgebrochende Schraube mühelos entfernt werden. Außerdem ragt ein Abschnitt 19 der abgebrochenen Schraube soweit über die Knochenoberfläche 20 hinaus, daß dieses Segment auch mit einem Betätigungswerkzeug, wie z.B. einer Zange zum Zwecke der Entfernung gefaßt werden kann.
- In Fig. 5: ist ein Osteosynthesenplattensegment 21 mit einem kugelsegmentartigem Rundloch 22 für den Schraubenkopf dargestellt, das bei 23 in den zylindrischen Durchsetzungsabschnitt 24 übergeht. Der Öffnungswinkel 25 beschreibt den Öffnungswinkel im Querschnittsbild, der den Übergang vom kugelsegmentartigen Auflagebereich für den Schraubenkopf zum Durchsetzungsbereich 24 charakterisiert, im dem der verstärkte Schraubenschaft liegt. Damit bei Biege- und Scherbelastungen keine den schraubenkopfabseitigen Abschnitt des Verstärkungsbereichs überlastenden Biegemomente auftreten, muß der Öffnungswinkel 25 über einem bestimmten Minimalbetrag liegen. Dadurch kann vermieden werden, daß durch ungünstig einwirkende Biegemomente auf den Verstärkungsbereich des Schraubenschaftes ein Bruch nahe der Knochenoberfläche auftritt. Durch die Vergrößerung des Öffnungswinkels kann durch die abgestimmte Schwächung des kritischen Schraubenlochbereichs hier eine Sollbruchstelle für das Gesamtimplantat eingerichtet werden.
- Fig. 6: zeigt eine gebrochene Schraube aus dem Stand der Technik zur Wirbelsäulenfixation, die aus einer ein Maschinengewinde aufweisenden Ankopplungseinrichtung 26, einem Knochengewindeabschnitt 27 und einer den Knochengewindeabschnitt im Durchmesser überragenden Handhabe 28 besteht. Aufgrund der geometrischen Verhältnisse kommt es bei Überlastungen zum Bruch im Knochengewindebereich 29, wobei dann das im Knochen liegende Schraubensegment keine Handhabe für den Angriff eines Betätigungswerkzeuges mehr aufweist und dieser Abschnitt, da im Knochen liegend, auch ohne Knochenresektion nicht gefaßt werden kann.

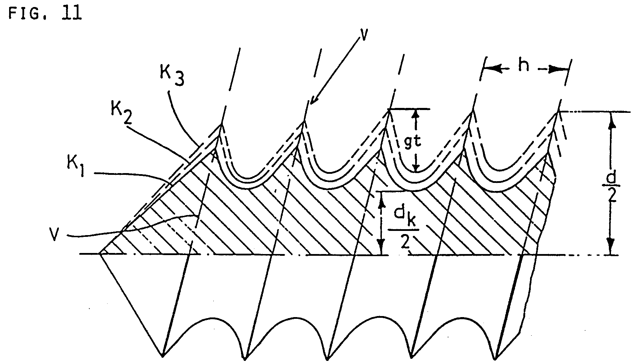

- Fig. 7: zeigt ein erfindungsgemäßes Ausführungsbeispiel einer Schraube für die Wirbelsäulenfixation, die wiederum einen Abschnitt 30 mit Maschinengewinde, eine Handhabe 31 und einen Knochengewindebereich 32 aufweist. Dieser Knochengewindebereich besteht aus einem ersten zylindrischen Abschnitt 33 und einem nicht-zylindrischen Verstärkungsbereich 34, der z.B. eine konische Außen- und Kernform aufweisen kann. An die Handhabe schließt auf der knochengewindeabseitigen Seite ein Bereich 35 an, in dem die Sollbruchstelle durch Querschnittsreduzierung ausgebildet ist. Am knochengewindeabseitigen Ende der Schraube ist eine zweite Handhabe 36 eingerichtet, über die eine Mutter 37 auf den Maschinengewindeabschnitt 30 verbracht werden kann. Das Innengewinde der Mutter 37 und/oder Außengewinde 30 des Schraubenschaftes können mit einem ein selbsttätiges Losdrehen verhinderndes Lockgewindeprofil ausgestattet sein.
- Fig. 8: stellt ein Segment einer anderen Ausführungsform der in den Schraubenschaft integrierten Ankopplungseinrichtung dar, die sich mit ihrem Schaft 38 an das knochengewindeabseitige Ende des Sollbruchsabschnitts anschließt. An ihrem anderen Ende befindet sich eine mit einer gerasterten Oberfläche ausgestattete Anlagerungsfläche 39, die mittels einer Schraube, die in die Gewindeöffnung 40 eingreift, auf eine zweite, ebenfalls gerasterte Scheibe angepreßt wird, wobei letztere in einen Lastträger integriert sein kann.
- In Fig. 9: ist ein Segment einer zur Wirbelsäulenfixation geeigneten Schraube 41 dargestellt, auf deren ein Maschinengewinde enthaltenden Ankopplungseinrichtung 42 eine zweiteilige Klammer 43/44 aufgeschoben ist. Das Klammersegment 43 weist an seiner Stirnseite eine sechskantige Öffnung 45 auf, in eine ebenfalls sechskantige Handhabe 46 rotationssicher einrasten kann. Wenn die Klammersegmente 43/44 mittels einer nachfolgenden Mutter 37 gegen die Stirnwand 47 des Verstärkungsbereichs, an die die Handhabe anschließt, angepreßt wird, kann eine rotationssichere Verbindung zu einem in die Hohlkehlen 48/49 der beiden Klammersegmenten eingelagerten, stabförmigen Lastträger geschaffen werden. Die Stirnwand 47 wie auch die Hohlkehlen 48/49 können eine gerillte, gerasterte, geriffelte oder aufgerauhte Oberflächenstruktur aufweisen, umso eine rotationssichere Verbindung mit den angelagerten Fixationselementen sicherzustellen. Am Ober- und/oder Unterrand 50 der Hohlkehlen einer oder beider Klammersegmente kann eine prominente Schneidkante eingearbeitet sein, die bei erfolgter Endmontage in die prominenten Oberflächenstrukturen eines stabförmigen Lastträgers eindringt und so eine ungewollte Längsverschiebung der Klammer auf dem stabförmigen Lastträger verhindert. Diese einer Einkerbung vergleichbare Einformung kann bei Überlastungssituationen wie eine Sollbruchstelle wirken, sodaß durch Bruch an dieser Stelle, Brüche der in den Knochen hineinreichenden Fixationsmittel vermieden werden können.
- Fig. 10: stellt einen stabförmigen Lastträger dar, der eine zylindrische Gestalt aufweist und an dem mittels einer segmentalen Querschnittsreduzierung 51 eine Sollbruchstelle ausgebildet ist.
An seiner Außenseite des Schaftes 52 weist der Lastträger eine Längsrillung 53 auf, die mit der gerillten Oberfläche der Hohlkehlen 48/49 der zweiteiligen Klammer in Fig. 9 in Kontakt tritt.
- Fig. 11: zeigt schematisch die Gewindeausbildung von drei unterschiedliche Außen- und Kerndurchmesser aufweisenden Knochenschraubenteilen. Die verschiedenen Knochenschraubenteile sind dabei mit K₁, ₂ und K₃ bezeichnet. Die Gewindesteigung ist mit dem Zeichen h versehen, der Außendurchmesser mit dem Zeichen d, der Kerndurchmesser mit dem Zeichen dₖ und die Gewindetiefe mit dem Zeichen gt.
Die Gewindegänge der drei Teilschrauben sind übereinander dargestellt und in der Zeichnung sind die Gewindescheitel der drei Teilschrauben K₁, K₂ und K₃ durch eine Verbindungslinie V verbunden. Diese Verbindungslinie liegt auf der Winkelhalbierenden der Gewindegänge der drei Schrauben.
Die ersten Gewindegänge einer Größe sind dem Aussendurchmesser der nächstkleineren Nenngröße angenähert und steigen dann zum Durchmesser der eigenen Nenngröße an.
Durch eine solche Ausgestaltung wird erreicht, daß bei Anwenden größenmäßig aufeinanderfolgender Gewindbohrer bzw. Knochenschrauben ein Verletzen der vorher vorhandenen Gewindegänge vermieden wird.

## Patentansprüche

1. Kraftübertragungsglied für Osteosynthesearbeiten in Form eines nur während einer Operationszeit in den Knochen eingreifenden Operationsinstruments, wie eines Knochengewindebohrers, eines Gewindeschneiders, einer flexiblen Welle, einer Fräse, bestehend aus einem mit dem Knochen kraftschlüssig in Verbindung tretenden Schaft und einer Ankopplungsvorrichtung an andere osteosynthetische Hilfsmittel, wobei das Kraftübertragungsglied eine im Anwendungsfall zwischen Ankopplungsvorrichtung und Knochen befindliche und außerhalb der Knochenmasse liegende Sollbruchstelle beinhaltet und im Falle des Bruches des Kraftübertragungsgliedes in der Sollbruchstelle ein die Knochenoberfläche überragendes und einen direkten Angriff eines Betätigungswerkzeuges ermöglichendes Segment aufweist.

2. Implantierbares Kraftübertragungsglied für Osteosynthesearbeiten bestehend aus einem mit dem Knochen kraftschlüssig in Verbindung tretenden Schaft (2, 21, 33, 52) und einer Ankopplungsvorrichtung (3, 22, 39, 38, 42, 44, 53) an andere osteosynthetische Hilfsmittel, dadurch gekennzeichnet, daß das Kraftübertragungsglied eine im Anwendungsfall nach seiner Befestigung im oder am Knochen zwischen Ankopplungsvorrichtung und Knochen befindliche und außerhalb der Knochenmasse liegende Sollbruchstelle (17, 22, 35, 51) beinhaltet und im Falle des Bruches des Kraftübertragungsgliedes in der Sollbruchstelle ein die Knochenoberfläche überragendes und einen direkten Angriff eines Betätigungswerkzeuges ermöglichendes Segment (19, 31, 52) aufweist.

3. Kraftübertragungsglied nach Anspruch 2, dadurch gekennzeichnet, daß zumindest ein Teil des Schaftes als ein in den Knochen eingreifendes Fixationsmittel, wie Knochenstift, gewindetragender Körper, Knochenschraube (32), Schraubeinsatz, Nagel, Draht, Prothesenkomponente, ausgebildet ist.

4. Kraftübertragungsglied nach Anspruch 2, dadurch gekennzeichnet, daß der Schaft Teil eines außerhalb des Knochens befindlichen oder ihm aufliegenden osteosynthetischen Implantats, wie einer Knochenplatte (21), eines Stabs (52), eines Rohrs, eines Drahts, einer Klammer, ist.

5. Kraftübertragungsglied nach einem oder mehreren der vorangehenden Ansprüche, dadurch gekennzeichnet, daß zumindest eine erste Handhabe (15, 31) für ein Betätigungswerkzeug eingearbeitet ist.

6. Kraftübertragungsglied nach Anspruch 5, dadurch gekennzeichnet, daß die Handhabe eine zur Druckausübung geeignete Stirnfläche (47) beinhaltet.

7. Kraftübertragungsglied nach Anspruch 5 oder 6, dadurch gekennzeichnet, daß eine zweite Handhabe (16, 36) für ein Betätigungswerkzeug eingearbeitet ist.

8. Kraftübertragungsglied nach einem der Ansprüche 5 bis 7, dadurch gekennzeichnet, daß zumindest eine Handhabe einen von der reinen Kreisform abweichenden Querschnitt (31) und ein Kraftübertragungs- und Antriebssegment aufweist.

9. Kraftübertragungsglied nach Anspruch 8, dadurch gekennzeichnet, daß zumindest eine Handhabe zwei Antriebssegmente (15, 16) umfaßt.

10. Kraftübertragungsglied nach Anspruch 9, dadurch gekennzeichnet, daß zumindest ein Antriebssegment eine konische oder sphärische Form aufweist.

11. Kraftübertragungsglied nach einem oder mehreren der Vorangehenden Ansprüche, dadurch gekennzeichnet, daß die Sollbruchstelle durch eine materialmäßige Schwächung, wie Einkerbung, segmentale Querschnittsreduzierung (35), Einschnürung (17), ausgebildet ist.

12. Kraftübertragungsglied nach Anspruch 2 oder einem oder mehreren der auf Anspruch 2 rückbezogenen, vorangehenden Ansprüche, dadurch gekennzeichnet, daR die Sollbruchstelle durch querschnittsmäßige Unterdimensionierung in einem außerhalb des Knochens befindlichen als Implantatkomponente (52) befindlichen Kraftübertragungsglied ausgestaltet ist.

13. Kraftübertragungsglied nach Anspruch 2 oder einem oder mehreren der auf Anspruch 2 rückbezogenen, vorangehenden Ansprüche, das mit anderen Kraftübertragungskomponenten ein aus mehreren Komponenten zusammengesetztes Implantatsystem bildet, dadurch gekennzeichnet, daß eine Implantatkomponente im Kontaktbereich zum Kraftübertragungsglied eine prominente Kante (50) aufweist, die bei der Montage am Schaft des Kraftübertragungsglieds (53) eine Kerbwirkung erzielt und dadurch eine Sollbruchstelle am Kraftübertragungsglied erzeugt.

14. Kraftübertragungsglied nach Anspruch 4, bei dem der Schaft als Teil einer Osteosyntheseplatte ausgebildet ist und zumindest ein Schraubenloch aufweist, das in einem Übergangsbereich (23) in einen Durchsetzungsabschnitt (24) einmündet, wobei die Normalen zu den Tangenten an die Flächen des Schraubenloches im Übergangsbereich (23) einen sich zum Durchsetzungsbereich öffnenden Winkel alpha (25) einschließen, dadurch gekennzeichnet, daß die Sollbruchstelle im Bereich des Schraubenlochs lokalisiert und der Öffnungswinkel alpha (25) größer als 75° ist.

15. Kraftübertragungsglied nach Ansprüchen 3 und 5 oder einem oder mehreren der auf diese Ansprüche rückbezogenen, vorangehenden Ansprüche, dadurch gekennzeichnet, daß die Sollbruchstelle zwischen dem Knochengewindeabschnitt und der Ankopplungseinrichtung so ausgebildet ist, daR beim Bruch des gewindetragenden Körpers im Bereich der Sollbruchstelle zumindest ein funktionsfähiges Segment (16) der Handhabe am Knochengewindesegment verbleibt.

16. Kraftübertragungsglied nach Ansprüchen 3 und 5 oder einem oder mehreren der auf diese Ansprüche rückbezogenen, vorangehenden Ansprüche, dadurch gekennzeichnet, daß der Schaft zumindest in einem Abschnitt ein Gewinde trägt und im Übergang vom Knochengewindeabschnitt zur Handhabe einen Verstärkungsbereich (14, 34) aufweist.

17. Kraftübertragungsglied nach Anspruch 16, dadurch gekennzeichnet, daß der Verstärkungsbereich eine von der Zylinderform abweichende Außenkontur, wie konisch (34), sphärisch oder sphäroidisch, aufweist.

18. Kraftübertragungsglied nach Anspruch 16 oder 17, dadurch gekennzeichnet, daß der Verstärkungsbereich durch eine Querschnittsvergrößerung des Außen- und/oder Kerndurchmessers charakterisiert ist.

19. Kraftübertragungsglied nach Ansprüchen 3 und 5 und 16 oder einem oder mehreren der auf diese Ansprüche rückbezogenen, vorangehenden Ansprüche, dadurch gekennzeichnet, daß ein Segment (16) der Handhabe bis in den Verstärkungsbereich (14) hineinreicht.

20. Kraftübertragungsglied nach Ansprüchen 3 und 16 oder einem oder mehreren der auf diese Ansprüche rückbezogenen, vorangehenden Ansprüche, dadurch gekennzeichnet, daß eine Handhabe (31) zwischen dem Verstärkungsbereich und der Ankopplungsvorrichtung plaziert ist.

21. Kraftübertragungsglied nach einem oder mehreren der vorangehenden Ansprüche, dadurch gekennzeichnet, daß eine Handhabe zumindest ein Teil der Ankopplungsvorrichtung ist.

22. Kraftübertragungsglied nach einem oder mehreren der vorangehenden Ansprüche, dadurch gekennzeichnet, daß ein Teil der Ankopplungsvorrichtung (47, 48, 49) eine profilierte Angriffsfläche, wie z.B. gerillt, gerastert, geriffelt, aufweist.

23. Kraftübertragungsglied nach einem oder mehreren der vorangehenden Ansprüche, dadurch gekennzeichnet, daß die Ankopplungsvorrichtung einen Innen- und/ oder Außengewindeabschnitt (30, 40) beinhaltet und zumindest eines der in den Ankopplungsbereich integrierten Gewinde ein selbsthemmendes Gewindeprofil aufweist.

24. Satz Kraftübertragungsglieder nach einem oder mehreren der vorangehenden Ansprüche, dadurch gekennzeichnet, daß die Kraftübertragungsglieder Knochengewinde tragen und jedes Kraftübertragungsglied des Satzes auch bei unterschiedlichen Nenndurchmesser (d) gleiche Steigung (h) aufweist.

25. Satz Kraftübertragungsglieder nach einem oder mehreren der vorangehenden Ansprüche, dadurch gekennzeichnet, daß die Kraftübertragungsglieder Knochengewinde tragen und jedes Kraftübertragungsglied des Satzes auch bei unterschiedlichen Kerndurchmessern (dₖ) gleiche Steigung (h) aufweist.

26. Satz Kraftübertragungsglieder nach einem oder mehreren der vorangehenden Ansprüche, dadurch gekennzeichnet, daß die Kraftübertragungsglieder Knochengewinde tragen und jedes Kraftübertragungsglied des Satzes auch bei unterschiedlicher Gewindetiefe (gt) gleiche Steigung (h) aufweist.

27. Satz Kraftübertragungsglieder nach einem oder mehreren der Ansprüche 24 bis 26, dadurch gekennzeichnet, daß sich der Satz zumindest aus je einem Operationsinstrument, wie z.B. eine Gewindebohrer und je einem größenmäßig dazu passenden Knochenimplantat, wie z.B. einer Knochenschraube, zusammensetzt.

28. Satz Kraftübertragungslieder nach Anspruch 27, dadurch gekennzeichnet, daß die Außen- und/oder Kerndurchmesser der Knochenimplantate 0,05 bis 1,0 mm über den korrespondierenden Durchmessern der Operationsinstrumente liegen.

## Claims

1. A load transfer member for osteosynthesis in the form of an operating instrument engaging in the bone only during an operation, such as a bone tap, a thread cutter, a flexible shaft or a milling cutter, consisting of a shank connecting non-positively with the bone and a device for coupling to other osteosynthetic aids, wherein the load transfer member comprises a predetermined breaking point located when in use between the coupling device and the bone and lying outside the bone mass and, in the case of breakage of the load transfer member at the predetermined breaking point, comprises a segment projecting beyond the bone surface and permitting direct engagement of an actuating tool.

2. An implantable load transfer member for osteosynthesis, consisting of a shank (2, 21, 33, 52) connecting nonpositively with the bone and a device (3, 22, 39, 38, 42, 44, 53) for coupling to other osteosynthetic aids, characterised in that the load transfer member comprises a predetermined breaking point (17, 22, 35, 51) located, when in use and after its attachment in or on the bone, between the coupling device and the bone and lying outside the bone mass and, in the case of breakage of the load transfer member at the predetermined breaking point, comprises a segment (19, 31, 52) projecting beyond the bone surface and permitting direct engagement of an actuating tool.

3. A load transfer member according to claim 2, characterised in that at least part of the shank is constructed as a fixing means engaging in the bone, such as a bone pin, a thread-bearing member, a bone screw (32), a screw insert, nail, wire or prosthesis component.

4. A load transfer member according to claim 2, characterised in that the shank is part of an osteosynthetic implant located outside the bone or lying thereon, such as a bone plate (21), a rod (52), a tube, a wire or a clip.

5. A load transfer member according to any one of the preceding claims, characterised in that at least a first handling element (15, 31) is formed for an actuating tool.

6. A load transfer member according to claim 5, characterised in that the handling element comprises an end face (47) suitable for the exertion of pressure.

7. A load transfer member according to claim 5 or claim 6, characterised in that a second handling element (16, 36) is formed for an actuating tool.

8. A load transfer member according to any one of claims 5 to 7, characterised in that at least one handling element has a cross section (31) deviating from the purely circular and a load transfer and driving segment.

9. A load transfer member according to claim 8, characterised in that at least one handling element comprises two driving segments (15, 16).

10. A load transfer member according to claim 9, characterised in that at least one driving segment is conical or spherical in form.

11. A load transfer member according to any one of the preceding claims, characterised in that the predetermined breaking point is formed by material weakening, such as notching, segmental cross-sectional reduction (35) or constriction (17).

12. A load transfer member according to claim 2 or any of the preceding claims dependent on claim 2, characterised in that the predetermined breaking point is formed by cross-sectional undersizing in a load transfer member located outside the bone and acting as an implant component (52).

13. A load transfer member according to claim 2 or any one of the preceding claims dependent on claim 2, said member forming, with other load transfer components, an implant system composed of several components, characterised in that an implant component comprises a prominent edge (50) in the contact area, which edge (50) has a notching effect on the shank of the load transfer member (53) during assembly and thereby generates a predetermined breaking point on the load transfer member.

14. A load transfer member according to claim 4, in which the shank is constructed as part of an osteosynthetic plate and comprises at least one screw hole, which opens into a passage portion (24) in a transition area (23), the line normal to the tangents on the faces of the screw hole in the transition area (23) forming an angle alpha (25) opening in the passage area, characterised in that the predetermined breaking point is located in the area of the screw hole and the aperture angle alpha (25) is larger than 75°.

15. A load transfer member according to claims 3 and 5 or any one of the preceding claims dependent on these claims, characterised in that the predetermined breaking point is formed between the bone thread portion and the coupling device in such a way that, when the thread-bearing member breaks in the area of the predetermined breaking point, at least one operative segment (16) of the handling element remains on the bone thread segment.

16. A load transfer member according to claims 3 and 5 or any one of the preceding claims dependent on these claims, characterised in that the shank is threaded at least over a portion thereof and comprises a widened area (14, 34) at the transition point from the bone thread portion to the handling element.

17. A load transfer member according to claim 16, characterised in that the widened area has an outer contour deviating from the cylindrical, for example conical (34), spherical or spheroidal.

18. A load transfer member according to claim 16 or claim 17, characterised in that the widened area is characterised by an increase in the cross section of the outer and/or core diameter.

19. A load transfer member according to claims 3, 5 and 16 or any one of the preceding claims dependent on these claims, characterised in that a segment (16) of the handling element extends into the widened area (14).

20. A load transfer member according to claims 3 and 16 or any one of the preceding claims dependent on these claims, characterised in that a handling element (31) is positioned between the widened area and the coupling device.

21. A load transfer member according to any one of the preceding claims, characterised in that a handling element is at least part of the coupling device.

22. A load transfer member according to any one of the preceding claims, characterised in that part of the coupling device (47, 48, 49) comprises a profiled engagement surface, such as, for example, a grooved, textured or corrugated surface.

23. A load transfer member according to any one of the preceding claims, characterised in that the coupling device comprises an inner and/or outer threaded portion (30, 40) and at least one of the threads integral with the coupling area comprises a self-locking thread profile.

24. A set of load transfer members according to any one of the preceding claims, characterised in that the load transfer members have bone threads and each load transfer member of the set has the same pitch (h) even when the nominal diameter (d) varies.

25. A set of load transfer members according to any one of the preceding claims, characterised in that the load transfer members have bone threads and each load transfer member of the set has the same pitch (h) even when the core diameters (dₖ) vary.

26. A set of load transfer members according to any one of the preceding claims, characterised in that the load transfer members have bone threads and each load transfer member of the set has the same pitch (h) even when the thread depth (gt) varies.

27. A set of load transfer members according to any one of claims 24 to 26, characterised in that each set is composed at least of one operating instrument, such as a tap for example, and a bone implant adaptable in size thereto, such as a bone screw for example.

28. A set of load transfer members according to claim 27, characterised in that the outer and/or core diameters of the bone implants are from 0.5 to 1.0 mm over the corresponding diameters of the operating instruments.

## Revendications

1. Elément de transfert de charge pour travaux d'ostéosynthèse sous la forme d'un instrument opératoire ne pénétrant dans l'os que durant le temps d'une opération, comme un taraud à os, un outil à fileter, un axe flexible, une fraise, composé d'une tige reliée par adhérence à l'os et d'un dispositif d'accouplement à d'autres moyens d'ostéosynthèse, où l'élément de transfert de charge contient en cours d'utilisation un point destiné à la rupture situé entre le dispositif d'accouplement et l'os et à l'extérieur de la masse osseuse et présente en cas de fracture de l'élément de transfert de charge au niveau du point destiné à la rupture un segment dépassant de la surface de l'os et rendant possible une prise directe pour un outil de manipulation.

2. Elément de transfert de charge implantable pour travaux d'ostéosynthèse comprenant une tige (2, 21, 33, 52) reliée par adhérence à l'os et un dispositif d'accouplement (3, 22, 39, 38, 42, 44, 53) avec d'autres moyens d'ostéosynthèse, **caractérisé en ce que** l'élément de transfert de charge contient en cas d'emploi après fixation dans l'os ou sur l'os un point destiné à la rupture (17, 22, 35, 51) situé entre le dispositif d'accouplement et l'os et placé hors de la masse osseuse et qu'il présente en cas de fracture de l'élément de transfert de charge au niveau du point destiné à la rupture un segment (19, 31, 52) dépassant de la surface osseuse et permettant une prise directe par un outil de manipulation.

3. Elément de transfert de charge selon la revendication 2, **caractérisé en ce que** au moins une partie de la tige est façonnée comme moyen de fixation pénétrant dans l'os, tel que broche, corps fileté, vis à os (32) pièce de vissage, clou, fil, éléments de prothèse.

4. Elément de transfert de charge selon la revendication 2, **caractérisé en ce que** la tige fait partie d'un implant d'ostéosynthèse situé à l'extérieur de l'os ou placé sur celui-ci, comme une plaque à os (21), une tige (52), un tuyau, un fil, une pince.

5. Elément de transfert de charge selon une ou plusieurs des revendications précédentes, **caractérisé en ce que** au moins une première prise (15, 31) est encastrée pour un outil de manipulation.

6. Elément de transfert de charge selon la revendication 5, **caractérisé en ce que** la prise comporte une face frontale (47) convenant à l'exercice d'une pression.

7. Elément de transfert de charge selon la revendication 5 ou 6, **caractérisé en ce que** une deuxième prise (16, 36) est encastrée pour un outil de manipulation.

8. Elément de transfert de charge selon une des revendications 5 à 7, **caractérisé en ce que** au moins une prise présente une coupe transversale (31) déviant de la forme purement circulaire et un segment de transfert de charge et d'entraînement.

9. Elément de transfert de charge selon la revendication 8, **caractérisé en ce que** au moins une prise englobe deux segments d'entraînement (15, 16).

10. Elément de transfert de charge selon la revendication 9, **caractérisé en ce que** au moins un segment d'entraînement présente une forme conique ou sphérique.

11. Elément de transfert de charge selon une ou plusieurs des revendications précédentes, **caractérisé en ce que** le point destiné à la rupture est façonné par un affaiblissement matériel, comme une entaille, une réduction de section de segment (35), un rétrécissement (17).

12. Elément de transfert de charge selon la revendication 2 ou une ou plusieurs des revendications précédentes dépendant de la revendication 2, **caractérisé en ce que** le point destiné à la rupture est façonné par un sousdimensionnement de la section d'un élément de transfert de charge situé hors de l'os comme élément d' implant (52).

13. Elément de transfert de charge selon la revendication 2 ou une ou plusieurs des revendications précédentes dépendant de la revendication 2, qui constitue avec d'autres composants de transfert de charge un système d'implant composé de plusieurs éléments, **caractérisé en ce que** un élément d'implant dans la zone de contact avec l'élément de transfert de charge présente un bord en saillie (50) qui vise un effet d'entaille lors du montage sur la tige de l'élément de transfert de charge (53) et crée par conséquent un point destiné à la rupture sur l'élément de transfert de charge.

14. Elément de transfert de charge selon la revendication 4, où la tige est façonnée comme une partie d'une plaque d'ostéosynthèse et présente au moins un trou de vis qui débouche dans une zone de transition (23) en une section traversante (24), où les normales aux tangentes des faces du trou de vis dans la zone de transition (23) entourent un angle alpha (25) s'ouvrant vers la zone traversante, **caractérisé en ce que** le point destiné à la rupture est localisé dans la zone du trou de vis et l'angle d'ouverture alpha (25) est supérieur à 75°.

15. Elément de transfert de charge selon les revendications 3 et 5 ou selon une ou plusieurs des revendications précédentes dépendant des dites revendications, **caractérisé en ce que** le point destiné à la rupture entre la section de taraudage de l'os et le dispositif d'accouplement est façonné de telle sorte que lors d'une fracture du corps fileté dans la zone du point destiné à la rupture au moins un segment fonctionnel (16) de la prise reste attaché au segment fileté dans l'os.

16. Elément de transfert de charge selon les revendications 3 et 5 ou selon une ou plusieurs des revendications précédentes dépendant des dites revendications, **caractérisé en ce que** la tige comporte au moins en une section un filetage et présente une zone renforcée (14, 34) à la transition entre la section de taraudage de l'os et la prise.

17. Elément de transfert de charge selon la revendication 16, **caractérisé en ce que** la zone renforcée présente un contour extérieur déviant de la forme cylindrique, comme conique (34), sphérique ou sphéroïde.

18. Elément de transfert de charge selon la revendication 16 ou 17, **caractérisé en ce que** la zone renforcée est caractérisée par une augmentation de section du diamètre extérieur et/ou intérieur.

19. Elément de transfert de charge selon les revendications 3 et 5 et 16 ou selon une ou plusieurs des revendications précédentes dépendant de ces revendications, **caractérisé en ce que** un segment (16) de la prise se prolonge jusque dans la zone renforcée (14).

20. Elément de transfert de charge selon les revendications 3 et 16 ou selon une ou plusieurs des revendications précédentes dépendant de ces revendications, **caractérisé en ce que** une prise (31) est placée entre la zone renforcée et le dispositif d'accouplement.

21. Elément de transfert de charge selon une ou plusieurs des revendications précédentes, **caractérisé en ce que** une prise est au moins une partie du dispositif d'accouplement.

22. Elément de transfert de charge selon une ou plusieurs des revendications précédentes, **caractérisé en ce que** une partie du dispositif d'accouplement (47, 48, 49) présente une surface d'attaque profilée, par ex. rainurée, crantée, striée.

23. Elément de transfert de charge selon une ou plusieurs des revendications précédentes, **caractérisé en ce que** le dispositif d'accouplement comporte une section de filetage interne et/ou externe (30, 40) et qu'au moins un des filetages intégrés dans la zone d'accouplement présente un profil fileté autobloquant.

24. Jeu d'éléments de transfert de charge selon une ou plusieurs des revendications précédentes, **caractérisé en ce que** les éléments de transfert de charge portent des tarauds à os et que chaque élément de transfert de charge du jeu présente le même pas (h) même pour des diamètres nominaux divers (d).

25. Jeu d'éléments de transfert de charge selon une ou plusieurs des revendications précédentes, **caractérisé en ce que** les éléments de transfert de charge portent des tarauds à os et que chaque élément de transfert de charge du jeu présente le même pas (h) pour des diamètres nominaux divers (dₖ).

26. Jeu d'éléments de transfert de charge selon une ou plusieurs des revendications précédentes, **caractérisé en ce que** les éléments de transfert de charge portent des tarauds à os et que chaque élément de transfert de charge du jeu présente le même pas (h) pour des profondeurs de pas divers (gt).

27. Jeu d'éléments de transfert de charge selon une ou plusieurs des revendications 24 à 26, **caractérisé en ce que** le jeu se compose au moins d'un instrument opératoire, par ex. d'un taraudeur, et au moins d'un implant osseux, par ex. d'une vis à os, de taille appropriée.

28. Jeu d'éléments de transfert de charge selon la revendication 27, **caractérisé en ce que** le diamètre extérieur et/ou intérieur des implants osseux dépasse de 0,05 à 1,0 mm le diamètre correspondant des instruments opératoires.
